# EUROPEAN PATENT APPLICATION

(11) **EP 4 778 542 A1**
(43) Date of publication of application: **22.07.2026**
(21) Application number: 25152137.3
(22) Date of filing: 15.01.2025
(51) Int. Cl.: A61L 27/36, A61L 27/50

(54) **TREATMENT OF PERICARDIUM**

(71) Applicant: P+F Products + Features GmbH, 1190 Wien (AT)
(72) Inventor: Agreli, Guilherme, Vienna (AT)
(74) Representative: Manitz Finsterwald Patent- und Rechtsanwaltspartnerschaft mbB

(57) **Abstract**

The present invention is directed at a method for treating biological tissue and a biological tissue obtained by the treatment method, and specifically at a method for treating biological tissue so as to suppress the calcification, risk of biofilm adherent over pericardium and strength reduction of the tissue due to treatment as well as to suppress immunogenic response or improve immunocompatibility of the biological tissue.

## Description

### FIELD OF THE INVENTION

The present invention is directed at a method for treating biological tissue and a biological tissue obtained by the treatment method, and specifically at a method for treating biological tissue so as to suppress the calcification, risk of biofilm adherent over pericardium and strength reduction of the tissue due to treatment as well as to suppress immunogenic response or improve immunocompatibility of the biological tissue.

### BACKGROUND OF THE INVENTION

Biological tissues are widely used to make prosthetic replacements for heart valves and blood vessels as well as for transcatheter heart valves. They are connective tissues comprising collagen as the main component. Among these tissues, bovine pericardium is one of the most widely employed.

Pericardial tissue is the sac surrounding the heart which provides a natural barrier to infection for the heart and prevents adhesion to the surrounding tissue. The pericardium also serves mechanical roles, for example, by preventing over dilation of the heart, maintaining the correct anatomical position of the heart, and regulating the pressure to volume ratio in the left ventricle during diastole. The structure of the tissue determines its behaviour under loading in both conditions physiologic to the pericardium and as a prosthetic device.

However, biological tissues obtained directly from a slaughterhouse, in particular porcine and bovine cadavers, begin to degrade immediately. Therefore, in order to be able to exploit biological tissues as clinical material this deterioration must be stopped. The aim is to prolong the material's original structural and mechanical integrity and remove or at least neutralize the antigenic properties attributed to these materials.

Methods typically concentrate on creating new additional chemicals bonds between the collagen molecules. These supplementary links reinforce the tissue to obtain a tough and strong but non-viable material that maintains the original shape of the tissue.

For this purpose, a biological tissue, such as e.g. bovine or porcine pericardium or a heart valve, is chemically treated to improve its mechanical performance and immunogenic properties, reduce thrombogenicity and degradation, preserve sterility, and prolong the allowable storage period.

Accordingly, it is of significant importance to select a method of treating a biological tissue used for implantation which makes the tissue be readily available to medical personnel with minimal preparation prior to surgery. This reduces the opportunities for error and also the implantation time.

Various studies are known in this context from the prior art, in which biological tissues were treated by different methods. Reference is made in this context to the following references:
US 2008 / 0102439 A1 is directed to the preparation or storage of biological tissues, in particular mammalian tissue, used for surgical implantation, wherein said preparation method comprises contacting the biological tissue with a non-aqueous treatment solution comprising a polyhydric alcohol, such as glycerol, and a C1-C3 alcohol, and removing a portion of the treatment solution from the solution-treated biological tissue. The tissue of the mammal is selected from the group consisting of pericardium, aortic and pulmonary roots and valves, tendons, ligaments, skin, dura and peritoneum.
US 2018 / 0133365 A1 introduced a preparation method of a dry animal-derived collagen fiber tissue material comprising the steps of rinsing of an animal-derived collagen fiber tissue material that has been treated with a crosslinking agent, immersion of the rinsed tissue material in a non-aqueous alcoholic solution for dehydration, successive immersion of the tissue material that has been dehydrated with the non-aqueous alcoholic solution in saccharide solutions of different gradients of concentrations for gradient dehydration, taking out and drying of the gradient dehydrated tissue material, hermetic packaging of the dried tissue material and sterilization.
US 2011 / 0300625 A1 describes a method of preparing a tissue, in particular a pericardium tissue, wherein the method comprises providing a section of tissue harvested from a mammalian organism, and causing osmotic shocking of the section of tissue by performing multiple rinses of the section of tissue with distilled water, and further rinsing the section of tissue with isopropyl alcohol and contacting the section of tissue with one of a formalin solution or a glutaraldehyde solution.
US 5 413 798 A discloses a process for preparing bovine pericard materials and the use of the materials thus prepared as transplants or implants in human medicine and veterinary medicine. In particular, said document describes the process for treating bovine pericard tissue comprising the steps of wet-chemical processing the pericard tissue, drying the pericard tissue and sterilizing the pericard tissue, wherein the wet-chemical processing comprises separating from the surface of said tissue any adherent fat and basal membrane, contacting said tissue with an aqueous alkaline solution, contacting said tissue with a solution of a metal-ion complexing agent containing disodium EDTA and contacting said tissue with an aqueous buffer solution having a pH of 4.5 to 6.0 and rinsing with water.

In another approach, WO 01 / 41828 describes a method for treating a biomaterial comprising contacting the biomaterial with an anticalcification treatment solution, said anticalcification treatment solution being selected from a group consisting of higher alcohol solutions, polyol solutions and polar aprotic organic solvent solutions.

Further, US 2001 / 0023372 A1 discloses a method of preparing a tissue component for dry storage comprising providing an animal tissue component, treating said tissue component with an aqueous treatment solution comprising a dimensional stabilizer such as glycerol or a derivative thereof, storing said treated tissue component in a container that is essentially free of liquid.

EP 2 582 411 A2 and WO 2011/160085 A2 describe a method for improving the performance of biological implants. The method comprises obtaining a fresh bioprosthetic tissue, fixing the tissue with a fixation agent comprising glutaraldegyde, treating the tissue with a bioburden reduction solution comprising formaldehyde, ethanol and Tween^{®}solution, treating with an oxidizing agent such as antigenic carbohydrate comprising a vicinal diol, treating the tissue with a capping agent comprising a primary amine or alcohol or an acid, treating with a stabilizing agent, drying the tissue, and sterilizing the tissue with ethylene oxide.

US 2019/001023 A1 refers to a treatment of biological tissue to resist calcification, and discloses a method of preparing calcification-resistant tissue comprising providing fresh biological tissue, cross-linking the tissue to produce fixed tissue, treating the fixed tissue with an alcohol, and treating the tissue with a polyol.

The article *"The effect of ethylenediaminetetraacetic acid on calcofic degeneration in bovine pericardium"* is directed to treating bovine pericardium with PBS and then with PBS/glutaraldehyde solution. After that the tissue is placed in PBS/EDTA solution and then oncemore rinsed in PBS/glutaraldehyde solution (Nehir Sucu at all, Heart and Vessels, vol. 19, no. 2, 1.03.2004, pages 89-93*).*

WO 2020 / 161243 A1 discloses a method of treating biological tissue comprising the steps of soaking of the biological tissue treated with a crosslinking agent with a saline solution, contacting the soaked biological tissue with an aqueous solution comprising Hydrogen Peroxide, contacting the biological tissue with an aqueous solution comprising PBS and EDTA, contacting the biological tissue with a solution comprising glycerol, ethanol and EDTA, and contacting the biological tissue with a glycerol solution.

Accordingly, each of the above mentioned patent applications have been made to develop a biological tissue which can be used as bioprosthetic devices that can be stored dry before it is used for clinical applications. However, there are certain disadvantageous associated with the above mentioned tissue preparation methods.

For example, upon implantation a biological tissue, especially aldehyde fixed tissue, is susceptible to the formation of degenerative calcific deposits. Calcification, in particular pathologic calcification, of soft biological tissues due to deposition of calcium phosphate mineral salts in an implanted tissue is undesirable and the deposition of the calcific deposits can have severe consequences on device performance. Calcification of implants can lead to stiffening, structural instability and ultimately to device failure.

Therefore, it is of significant importance to provide biological tissues having resistance to calcification, especially because heart valve diseases necessitate valve replacement surgery in over several hundred thousand people worldwide each year.

Several treatments, such as mentioned above, were tested in order to eliminate or retard the calcification, but no method is as effective as desired. The decellularization is proposed as an alternative during the biological tissue processing of e.g. cardiovascular prostheses. The removal of cellular elements does not only reduce the tissue antigenicity, but it also potentially inhibits or slows down the calcification for eliminating the initial outbreaks of calcium deposits in membranes and cellular debris. Therefore, the decellularization process is expected to reduce or eliminate the calcification. Further, the decellularization can be proposed as a method to diminish tissue antigenicity leading to better tissue compatibility, remodelling and long-term durability.

The majority of clinically available bioprosthetic heart valves are composed of stent-mounted glutaraldehyde-fixed bovine pericardial tissue. Glutaraldehyde fixation effectively crosslinks the collagen in the tissue and to a great extent eliminates the immunogenicity and thrombogenicity of the bioprosthesis. However, a large amount of bioprosthetic heart valves fail due to pathologic calcification of the valve causing tissue stiffening and tearing.

Accordingly, it is desirable to provide a new anticalcification approach with improved efficacy and ease of use. Thus, there is a need for an effective method of imparting anticalcification properties to biological tissues used for surgical implantations that is not accompanied by deleterious effect and also provides improved mechanical characteristics of a biological tissue during its storage.

### SUMMARY OF THE INVENTION

For this reason it is an object of the present invention to provide a new method for preparing biological tissues that makes available biological tissues for surgical implantations in as close to ready-to-use form as possible. It is a further object of the present invention to provide a method of treating biological tissue which reduces calcification of the biological tissue, risk of biofilm adherence over pericardium and that provides improved mechanical properties to a bioprosthesis comprising the biological tissue. Another object of the present invention is to provide a method of treating a biological tissue, capable of avoiding and preferably preventing paravalvular leakage (PVL) as far as possible, which further results in a reduction of trauma during surgery, so that the stent can also be used with elderly patient.

These objects are satisfied by a respective method comprising the respective features of the independent claims. Specific embodiments of the invention are described in the dependent claims.

In this connection, in a first aspect of the present invention, a method of treating biological tissue is provided, comprising the steps of:
(1) contacting the biological tissue with an aqueous solution of Hydrogen Peroxide;
(2) contacting the biological tissue with an aqueous solution of Sodium Hydroxide;
(3) contacting the biological tissue with an aqueous solution of Triton, SDS and EDTA;
(4) contacting the biological tissue with a glycerol solution.

In this way a biological tissue is prepared for surgical implantations that is in as close to ready-to-use form as possible. Moreover, such a biological tissue has reduced calcification, a reduced risk of biofilm adherence over pericardium and improved mechanical properties. Further, such a treatment method allows preparing biological tissues with inactivated prions and viruses which sufficiently reduces the risk for a patient after the surgical implantation of this tissue. Moreover, such a biological tissue, is capable of being used in a stent that can avoid and preferably prevent paravalvular leakage (PVL) as far as possible. This further results in a reduction of trauma during surgery, so that the stent can also be used with elderly patients.

Preferably, the biological tissue is fixed with glutaraldehyde before treating, for example having a concentration selected in the range of 0.1% to 5.0% by volume. In this way the biological tissue is treated with a cross-linking agent. Such a cross-linking agent is used to chemically stabilize the biological tissue in order to avoid and preferably prevent a degradation of the collagenous tissue upon implantation into a host recipient.

In one embodiment, the method further comprises steps (2a) and/or (3a) of contacting the biological tissue with an aqueous solution of TRIS and EDTA. The use of such an aqueous solution has a significant influence on the reduction of the calcium content, i.e. leads to a reduction of the calcification of the biological tissue such as bovine pericardium membrane.

Preferably, the method further comprises steps (3b) and/or (4a) of contacting the biological tissue with ethanol within a concentration of at least 70% by volume. The ethanol treatment inhibits bioprosthesis calcification through an interaction of membrane-lipid removal and ethanol-induced collagen structural changes. Because cell membrane-oriented calcification and collagen calcification are the prominent pathophysiological features of bioprosthetic tissue mineralization, inhibition of both processes may be a prerequisite for high therapeutic efficacy. Furthermore, the high concentration of the ethanol used in the method was found to be more effective for such a purpose than the lower concentrated ethanol. Moreover, ethanol treatment results in no significant alterations in the biological tissue material characteristics and morphology.

It is preferred that the biological tissue according to the claimed method is bovine or porcine pericardium or a heart valve. These types of tissue are commonly used for surgical implantations as having superior intrinsic biological properties for e.g. prosthetic valve manufacture.

It is preferred that the concentration of hydrogen peroxide (H₂O₂) in step (1) according to the claimed method is from 1.0% to 30.0% by volume. In this connection it should be noted that the hydrogen peroxide is generally used as a mild oxidizing agent, which serves the purpose of oxidatively destroying any accompanying substances in the collagen without attacking the collagen itself. In this way hydrogen peroxide can e.g. sufficiently inactivate prions and viruses contained in the biological tissue thereby reducing the risk for a patient.

Preferably, sodium hydroxide (NaOH) in step (2) has a concentration of 0.1 M to 10M. The use of sodium hydroxide allows achieving a good cleaning effect of the biological tissue, which includes inactivating enzymes and potential germs as completely as possible, but on the other hand also prevents damage to the collagen-containing tissue. Further, the treatment with sodium hydroxide induces osmotic swelling of the tissues and reduction of the tissue shrinkage. The action of alkali metal on the collagen is represented by amino acids modification and destruction of intra-and intermolecular collagen cross-links.

It should also be noted that using NaOH within a well-controlled concentration range (from 0.1 M to 10 M) effectively cleans and disinfects biological tissues, as it inactivates enzymes and eliminates microorganisms.

Such a cleaning process also promotes osmotic swelling of fibers, which helps reduce tissue shrinkage. Regarding collagen integrity, it is true that alkaline solutions can modify amino acids and break intra- and intermolecular bonds, but, if the process is well managed-with careful selection of concentration, treatment time, and temperature-it is possible to avoid significant damage to the collagen matrix. Therefore, the statement is fundamentally correct, provided that proper processing parameters are established to prevent excessive collagen degradation.

It is preferred that in steps (2a) and (3a) a concentration of TRIS is of 1 mM to 30mM and a concentration of EDTA is of 0.1 mM to 5mM. The use of a mixture of TRIS and EDTA allows the prevention of degradation of nucleic acids. Further, the EDTA which is a chelating agent is used for elimination of contaminating divalent cations and inhibiting protease activity.

It is further preferred that in step (3) a concentration of Triton is of 0.1% to 10.0% by volume, a concentration of SDS is of 0.05% to 5.0% by volume and a concentration of EDTA is of 0.05mM to 5mM. The use of a mixture of Triton, SDS and EDTA provides a significant effect on removing all cellular and nuclear matter from the biological tissue, and also minimize any adverse effects on the composition, biological activity, and mechanical integrity of the remaining extracellular matrix.

Preferably, the treatment is carried out on the biological tissue which is fixed on a metallic frame. The metallic frame usually being a stent. The metallic frames or stents have advantages over e.g. plastic (polymer) stents, because they are less thick, do not prone to inflammation or fractures and have longer a storage shelf-life and are less likely to fail when used in a constantly contracting and expanding organ such as a human heart.

It is further preferred that the method further comprises the step (5) of drying the biological tissue. The drying process is within the skill in the art and the resulting product present in a substantially dry form.

Further preferably, the biological tissue is a heart valve for placement at a stent. The use of the stent is beneficial, because such a use allows preparing an anatomically tailored shape of the patient's native pathological heart valve thereby improving its function and lifespan.

Preferably, the step (5) of drying is carried out after fixing the heart valve to the stent. That means that the resulting product comprises the stent with a dry heart valve made e.g. from bovine pericardium, which upon final replacement at the point of interest can be rehydrated with a solution such as saline solution. The benefits of the fixation before treating is that the following implantation of the heart valves is more technically demanding.

It is preferred that the heart valve is attached to the stent prior to step (1). The beneficial effect of the attaching prior treating is that the wall portion of stanted valves calcified less than of stentless valves.

Regarding the assertion that "stented valves calcify less than stentless valves" when the stent is attached prior to decalcification or other treatments, it is plausible that the presence of the stent, combined with specific fixation and treatment protocols, could reduce mechanical stress or facilitate the action of anti-calcification agents, thereby helping to lower the risk of calcification.

In short, attaching the valve to the stent before drying (and before or during certain treatment steps) offers practical benefits and can indeed reduce calcification.

It is further preferred that the method of the present invention further comprises the step of testing the valve functionality prior to step (1). The testing of the biological tissue or a heart valve before treating allows providing an information and evidence for its continued structural integrity as well as for evaluating a heart valve fatigue.

Preferably, the method of treating biological tissue is a method of decellulizing the biological tissue. In particular, the decellulizing or decellularization of the biological tissue is a generalization of a series or a combination of treatment the biological tissue that ideally removes all cellular and nuclear materials without adversely affecting the composition, organization, biological activity, and mechanical integrity of the biological tissue. The efficiency of decellularization is usually a factor of both the processing methodology and the nature of the tissue.

A second aspect of the present invention comprises a method of treating biological tissue comprising the steps of:
(1) providing bovine or porcine pericardium as the biological tissue as a heart valve;
(2) providing a stent frame;
(3) attaching the biological tissue to the stent frame;
(4) testing the functionality of the biological tissue at the stent frame; and
(5) carrying out a method of decellulizing the biological tissue after step (4).

Particularly with regard to the above method, biological tissue can be made available at a stent frame that is capable of avoiding and preferably preventing paravalvular leakage (PVL) as far as possible once implanted into a patient.

Moreover, such a stent can be used in surgery and results in a reduction of trauma during the surgery, so that the stent can also be used with elderly patients.

Moreover, such a stent frame enables the provision of a bioprosthesis having improved mechanical properties.

It is preferred that the method of decellulizing the biological tissue comprises the steps of:
(1) contacting the biological tissue with an aqueous solution of Hydrogen Peroxide;
(2) contacting the biological tissue with an aqueous solution of Sodium Hydroxide;
(3) contacting the biological tissue with an aqueous solution of Triton, SDS and EDTA;
(4) contacting the biological tissue with a glycerol solution.

In a third aspect, the present invention relates to a biological tissue comprising a biological tissue prepared according to a claimed method, in particular to the method according to the first aspect or to the method according to the second aspect of the present invention.

Also with regard to this aspect a biological tissue is prepared for surgical implantations that is in as close to ready-to-use form as possible. Moreover, such a biological tissue has reduced calcification, a reduced risk of biofilm adherence over pericardium and improved mechanical properties. Moreover, such a biological tissue, is capable of being used in a stent that can avoid and preferably prevent paravalvular leakage (PVL) as far as possible. This further results in a reduction of trauma during surgery, so that the stent can also be used with elderly patients.

### BRIEF DESRIPTION OF THE DRAWINGS

Fig. 1: Calcification of test bovine pericardium samples of examples 1-1 to 1-3.
Fig. 2: Scanning Electron Microscope images (SEM) of the bovine pericardium: (a) and (c) without SBF, (b) and (d) immersed in SBF solutions for 24 hours.

### DETAILED DESCRIPTION OF THE INVENTION

In a first aspect, the present invention is directed at a method of treating biological tissue. Preferably, the method provides biological tissue containing such treated tissue in ready-to-use form for surgical implantations.

The term "biological tissue" is used herein to refer generally to treated or untreated collagen-containing biologically-derived human or animal materials.

The biological (animal) tissues are widely used to make prosthetic replacements for heart valves and blood vessels as well as for transcatheter heart valves. They are connective tissues comprising collagen as the main component. In the present invention, it is preferably to use a pericardium or a heart valve as an animal biological tissue material, in particular obtained from a porcine or bovine heart, that has been treated with the crosslinking agent. The natural human heart valves are identified as the aortic, mitral, tricuspid and pulmonary valves. Pericardium tissue or heart valve tissues are used to replace damaged or diseased heart valves and to replace any of the above mentioned naturally occurring valves.

In this respect, the biological tissues produced according to the inventive method are used for construction of a "bioprosthetic device" or a "bioprosthesis" having a function much like a natural human heart valve, with the bioprosthetic imitating the natural action of the flexible heart valve leaflets. Tissue type heart valves are also significantly difficult and time consuming to manufacture, because they must be manufactured to exact standards and tolerances in order to function for years within the dynamic environment of a living patient's heart.

The primary component of the biological tissues is collagen. Collagen molecules consist of three chains of polyamino acids arranged in a trihelical configuration ending in non-helical carboxyl and amino termini. These collagen molecules assemble to form microfibrils, which in turn assemble into fibrils, resulting in collagen fibers. Because collagenous tissues degrade rapidly upon implantation into a host recipient, it is necessary to stabilize the tissue if it is to be used for clinical applications. Chemical stabilization by tissue cross-linking, also known as tissue fixation, has been achieved using a variety of compounds.

Most typically, chemical fixation has employed polyfunctional molecules having two or more reactive groups capable of forming irreversible and stable intramolecular and intermolecular chemical bonds with the reactive amino acid side groups being present on the collagen molecules.

The most widely used of these polyfunctional molecules is glutaraldehyde, which has an aldehyde at each end of a linear aliphatic chain. The aldehyde groups of glutaraldehyde and other like molecules react under physiological conditions with the primary amine groups of collagen molecules to cross-link the material. Glutaraldehyde cross linked tissue produced in this way exhibits increased resistance to enzymatic degradation, reduced immunogenicity and increased stability.

Despite its widespread use, there are certain disadvantages associated with tissue crosslinking with glutaraldehyde. For example, upon implantation, aldehyde fixed tissue is susceptible to the formation of degenerative calcific deposits, i.e. calcification. Calcification is the undesirable deposition of calcium phosphate mineral salts in an implanted tissue and is the most significant factor of failure of glutaraldehyde-fixed biological tissues used as bioprosthetic devices.

The biological tissues obtained from the slaughterhouse, in particular porcine and bovine cadavers, begin to degrade immediately. Therefore, the storage of such materials has proven to be difficult. For this purpose, a biological tissue, such as e.g. bovine or porcine pericardium or a heart valve, is usually chemically treated to improve its mechanical performance and immunogenic properties, reduce thrombogenicity and degradation, preserve sterility, and prolong the allowable storage period.

Therefore, the present invention is directed at treating the biological tissue with specific solutions step by step as described below.

One embodiment of the present invention utilized contacting the biological tissue with an aqueous solution of Hydrogen Peroxide. The biological tissue is preferably treated with a crosslinking agent before treating with an aqueous solution of Hydrogen Peroxide.

As used herein, a crosslinking agent is glutaraldehyde which is preferably used in biochemical and medicine applications as an amine-reactive homobifunctional crosslinker. As already mentioned above, glutaraldehyde treatment produces stable cross-links in cellular and extra-cellular matrix proteins which substantially reduced graft immunogenicity.

In the present invention, it is preferably to use the crosslinking agent in an amount of from 0.1% to 5.0 % by volume, more preferably from 0.2% to 3.0% by volume, further preferably from 0.3% to 2.0% by volume and especially preferably from 0.5% to 1.0% by volume.

Prion diseases are a group of progressive and ultimately fatal neurodegenerative disorders caused by a proteinaceous infectious prion that can affect both humans and animals. The major component of the pathogen is the abnormal isoform of the prion protein. Human prion diseases may be caused by infection or inheritance, but most types of human prion disease are of unknown origin and are referred to as sporadic Creutzfeldt-Jakob disease (CJD). All types of prion disease are transmissible. Representative animal prion diseases include scrapie in sheep and goat, bovine spongiform encephalopathy (BSE) in cattle, and chronic wasting disease (CWD) in cervids. In terms of inactivation, prions are acknowledged to be the most resistant type of pathogen. Therefore, it is of significant importance to eliminate any prions from the biological tissue before the use as e.g. bioprosthetic device. Further, the treating of the biological tissue with an aqueous solution of Hydrogen Peroxide with a following treating with an aqueous solution of Sodium Hydroxide can sufficiently inactivate prions contained in the biological tissue thereby reducing the risk for a patient.

In this respect, as a first step a treating the biological tissue with an aqueous solution of Hydrogen Peroxide is carried out. It is preferred that the concentration of Hydrogen Peroxide is from 1.0% by volume to 30.0% by volume, preferably from 2.0% by volume to 20.0% by volume, more preferably from 1.0% by volume to 10.0% by volume, and most preferably from 3.0% by volume to 9.0% by volume.

It is preferably to treat biological tissue in a solution comprising Hydrogen Peroxide for at least 30 minutes, preferably for at least 60 minutes, more preferably for at least 90 minutes, at a temperature from 1°C to 10°C, in particular at a temperature of 2°C to 8°C, under stirring of not more than 500 rpm, preferably of not more than 300 rpm, more preferably of not more than 200 rpm, but not less than 50 rpm, preferably not less than 80 rpm, more preferably not less than 100 rpm.

In a second step of the present invention, the biological tissue is contacted with an aqueous solution of Sodium Hydroxide. It is preferred that the concentration of Sodium Hydroxide is from 0.1M to 10.0M, preferably from 0.2M to 8.0M, more preferably from 0.5M to 5.0M, most preferably from 0.8M to 2.0M. It is further preferably that pH of the aqueous solution of Sodium Hydroxide is from 10 to 14, preferably from 12 to 14, more preferably from 12.5 to 14.

It is preferably to treat biological tissue in a solution comprising Sodium Hydroxide for at least 30 minutes, preferably for at least 60 minutes, more preferably for at least 90 minutes, at a temperature from 1°C to 10°C, in particular at a temperature of 2°C to 8°C, under stirring of not more than 500 rpm, preferably of not more than 300 rpm, more preferably of not more than 200 rpm, but not less than 50 rpm, preferably not less than 80 rpm, more preferably not less than 100 rpm.

As used herein, the term "contacting" means treating, immersion, exposing to, rinsing of the biological tissue used in the inventive method.

The term "aqueous solution" refers to a solution comprising a substance or a compound and water that has been purified to remove contaminants which are able to influence the end product. Preferably, distilled water, double distilled water or deionized water is used in a method of the present invention.

In a third step of the method of the present invention, the biological tissue is contacted with an aqueous solution of Triton, SDS and EDTA.

The term "EDTA" is used herein to refer to ethylenediaminetetraacetic acid which is a complexing chelating agent being able to sequester metal ions especially like Fe²⁺/Fe³⁺, Al³⁺, Mg²⁺, Ca²⁺, Mn²⁺, Zn²⁺ and others and to remove them from the solution forming so called EDTA-complexes.

The term "Triton" is used herein to refer to "Triton X-100" which is 2-[4-(2,4,4-tri-methylpentan-2-yl)phenoxy]ethanol. It is a nonionic surfactant that has a hydrophilic polyethylene oxide chain and an aromatic hydrocarbon lipophilic or hydrophobic group.

The term "SDS" refers to SDS Solution which is solution of sodium dodecyl sulfate in distilled, deionized water. SDS is a detergent that is known to denature proteins. Preferably, SDS is used as 10% SDS solution which is filtered and ready to use.

It is preferred that the concentration of Triton in a solution containing a mixture of Triton, SDS and EDTA is from 0.1% to 10.0% by volume, preferably from 0.5% to 5.0% by volume, more preferably from 0.6% to 3.0% by volume, most preferably from 0.8% to 2.0% by volume.

It is further preferably that the concentration of SDS in the solution 0.05% to 5.0% by volume, preferably from 0.1% to 3.0% by volume, more preferably from 0.3% to 1.0% by volume. The concentration of EDTA in the solution is from 0.05mM to 5.0mM, preferably from 0.1mM to 3.0mM, more preferably from 0.3mM to 1.0mM.

According to the present invention, the method of treating biological tissue is a method of decellulizing the biological tissue. It is especially important to carry out the decellularization in a delicate balance, since extreme treatments run the risk of degrading extracellular matrix (ECM) structure and compromising the mechanical integrity of the tissue, while mild treatments will leave antigenic cellular components behind that might induce an immune response in the recipient. The end result of an optimal decellularization is a nonimmunogenic vessel scaffold with comparable extracellular matrix and mechanical characteristics comparable to native vessels. Therefore, it is very important to provide the most effective method for the decellularization of the biological tissues which can reduce risk of graft rejection or disease transmission.

The choice of the reagents is of critically importance, because they can damage the microstructure and composition of the resultant scaffold and thus, indirectly, may affect the mechanical properties of the ultimate product. Usually Triton, SDS and EDTA are used in separate steps in the treatments of the biological tissues. However, it has been found that a treatment using a mixture of Triton, SDS and EDTA provides the greatest effect on removing all cellular and nuclear matter from the biological tissue, and also minimize any adverse effects on the composition, biological activity, and mechanical integrity of the remaining extracellular matrix.

It is preferably to treat biological tissue in a solution comprising Triton, SDS and EDTA for at least 20 hours, preferably for at least 24 hours, more preferably for at least 36 hours, most preferably for at least 48 hours, at a temperature from 1°C to 10°C, in particular at a temperature of 2°C to 8°C, under stirring of not more than 500 rpm, preferably of not more than 300 rpm, more preferably of not more than 200 rpm, but not less than 50 rpm, preferably not less than 80 rpm, more preferably not less than 100 rpm.

In a fourth step of the method of the present invention, the biological tissue is contacted with a glycerol solution in order to dehydrate the biological tissue.

According to the present invention, the method further comprises steps (2a) and/or (3a) of contacting the biological tissue with an aqueous solution of TRIS and EDTA.

The term "TRIS" is used herein to refer to tris(hydroxymethyl)aminomethane. It is an organic compound which extensively used as a component of buffer solutions, in particular together with EDTA.

According to the present invention, it is especially important to remove calcium ions from the solution by forming calcium chelator that has been shown to inhibit mineralization of biological tissues, in particular bovine or porcine pericardium tissue. It is suggested that EDTA binds to calcium ions on the outer shell of hydroxyapatite crystals which are formed from calcium phosphate crystals thereby chelating and removing calcium ions from the crystals, causing the tissue material to shrink thus demineralizing the material.

Treatment of biological tissues with EDTA hence slows down the progression of calcification by binding calcium before it can react to form hydroxyapatite. Since the calcification of biological tissues used e.g. as bioprosthetic heart valves is a clinically significant problem that contributes to implant failure, it is of significant importance to reduce calcium level in biological tissues used as an implant.

Therefore in the present invention, the EDTA treatment can reduce calcium level in biological tissues, especially in bovine or porcine pericardium or a heart valve preferably by 20%, more preferably by 30%, further preferably by 40% and especially preferably by 50%. [A sample treated according to the present invention can show a calcium content of about 17 mg Ca²⁺ per gram of tissue, whereas a differently treated (control) sample may exhibit about 52mg Ca²⁺ per gram of tissue-i.e., a 33% reduction in calcium level.

Further, it is preferable to use EDTA in combination with TRIS in order to increase demineralization and compatibility with a human body.

It is preferred that the concentration of TRIS in a solution containing a mixture of TRIS and EDTA is from 1mM to 30mM, preferably from 2mM to 20mM, more preferably from 5mM to 15mM, most preferably from 8 mM to 12 mM. The concentration of EDTA in the solution is from 0.10mM to 5.0mM, preferably from 0.15mM to 3.0mM, more preferably from 0.20mM to 1.0mM, most preferably from 0.20mM to 0.50mM.

After the biological tissues have been processed through steps (3a) and/or (4) of the method of the present invention, they undergo preferably the treatment with ethanol.

Phospholipids in and around biological tissue cells have been found the most prominent calcification nucleation sites. Therefore, the removal of these tissue components has been proposed to reduce mineralization, in particular calcification.

The organic solvents like ethanol (steps 3b and/or 4a) or glycerol (step 4) can be similarly used for this purpose in the present application. For example, the treatment with at least 70% ethanol, preferably with at least 80% ethanol, more preferably with at least 90% ethanol, extracts phospholipids from the tissue while also causing a change in collagen conformation that increases bioprosthesis resistance to collagenase. Thus, ethanol treatment allows extracting almost all phospholipids and cholesterols from the bioprosthesis, thus eliminating calcification of the biological tissue cells.

Additionally, ethanol treatment also prevents adsorption of phospholipids and cholesterols from the solution. The method by which glycerol fixes biological tissue is not jet fully understood, but a 98% concentration, preferably 99% concentration, is sufficient to treat the biological tissue to make the tissue more biocompatible and resistant to calcification.

The biological tissues are removed from the solution and exposed to ambient air or an inert environment, e.g. nitrogen, at room temperature and humidity so as not to adversely affect tissue properties. Preferably, the drying is performed in a clean room at ambient conditions for at least 12 hours, preferably for at least 16 hours, still preferably for at least 20 hours.

Further preferably, the drying is performed under high efficiency particulate air (HEPA) filter, in particular under HEPA conditions in a clean room.

As used herein, the term "ambient conditions" is directed to the ambient temperature of more than 10°C, preferably of more than 12°C, more preferably of more than 14°C, especially preferably of more than 18°C, and preferably of less than 25°C, more preferably of less than 23°C, further preferably of less than 22°C. Further in the present invention it is preferably to carry out each of steps of the method of the present invention at the ambient conditions as described above.

Preferably, the dry biological tissue, e.g. dry bovine pericardium, has a calcium content selected in the range of 0.01 g/Kg and 0.1 g/Kg, preferably in the range of 0.015 to 0.09 g/Kg, in the range of 0.02 to 0.05 g/Kg.

Treating the biological tissues such as valves with a specialized anti-calcification treatment makes them more resistant to calcification. The valves having such a calcium content are hence more resistant to calcification and are more durable.

The treated and dried biological tissues are then preferably packaged in a container or package essentially free of liquid for subsequent surgical implantation. As used herein, the term "essentially free of liquid" means a non-fluid environment in which the presence of water or other substances is limited to approximately the content of such substances in ambient air.

One preferred method is to apply a vacuum to the package to minimize the level of oxygen, and one may additionally utilize a backfill of an inert gas, such as nitrogen. Such methods of sterile packaging are known to those skilled in the art.

The packaged treated tissue can then be sterilized by a gaseous sterilization process or by an exposure to ionizing radiation. To ensure that the chamber remains sterile following sterilization, the package members are formed from a material that is impenetrable to micro-organisms such as bacteria and fungi. Preferably, after the tissue or bioprosthetic device containing such tissue is placed in the chamber and/or sterilized, the chamber is sealed.

Sterilization by exposure to ionizing radiation or sterilizing gas, particularly by exposure to ethylene oxide gas, is within the skill of the art. In a preferred embodiment, biological tissues are sterilized by exposing the biological tissue to sterilizing gas, in particular to ethylene oxide gas (ETO). Sterilizing with ETO method is usually carried out at a temperature between 37°C and 55°C, gas mixture comprising at least 10% of ethylene oxide and for at least 60 minutes.

The resulting product is a substantially sterile and sealed implantable tissue present in a substantially dry form. It is especially well-suited for surgical implantation into human patients for the treatment of any number of diseases or conditions. Prior to surgical implantation, the biological tissue is removed from the package, and the tissue component optionally rehydrated by exposure to an aqueous solution, preferably a sterile aqueous solution. The tissue can be rehydrated by multiple soakings in a sterile solution such as physiologic saline. The glycerol or ethanol in the tissue can be easily washed off by saline.

The described method provides a biological tissue having dimensional stability and which is essentially ready for surgical implantation into a patient, i.e. so called ready-to-use form.

Biological tissues treated in accordance with the method of the present invention will typically return to a size that is at least 95%, more preferably at least 97%, further preferably at least 99% of its original hydrated size. As a result, the biological tissues prepared in accordance with the inventive method are ready-to-use for surgical implantation as an implantable bioprosthetic device, in particular in transcatheter heart valves.

Further in the present invention it is preferably to use a pericardium or a heart valve as a biological tissue. They are connective tissues comprising collagen as the main component. Accordingly, the pericardium or heart valve tissue can be processed according to the method of the present invention prior to their use for surgical implantation. Still further in the present invention it is preferable to use a pericardium or a heart valve obtained from an animal tissue, in particular from a porcine or bovine heart.

The bovine pericardium is one of the most widely employed tissues for heart valves. Pericardial tissue is the sac surrounding the heart which provides a natural barrier to infection for the heart and prevents adhesion to the surrounding tissue. The pericardium also serves mechanical roles, for example, by preventing over dilation of the heart, maintaining the correct anatomical position of the heart, and regulating the pressure to volume ratio in the left ventricle during diastole. The structure of the tissue determines its behaviour under loading in both conditions physiologic to the pericardium and as a prosthetic device. Preferably, the bovine pericardium obtained from a certified abattoir undergoes the procedures of cleaning, trimming and crosslinking with glutaraldehyde before the use in a method of the present invention.

The biological tissue prepared by the method of the present invention can be used as a bioprosthesis and transcatheter heart valves. As used herein, the term "bioprostheses" means a device derived from processed biological tissue to be used for implantation into humans. The development of such devices originated as an attempt to circumvent some of the clinical complications associated with the early development of the mechanical heart valve, and has since resulted in a rapid proliferation of bioprosthetic devices for a variety of applications. Examples of some of the bioprostheses currently used or under development include heart valves, vascular grafts, biohybrid vascular grafts, ligament substitutes, pericardial patches and others.

The most common types of animal tissue valves used include porcine aortic valves, and bovine and porcine pericardial valves (pericardium), some of which are incorporated with some type of a frame before implantation in a patient. In this case, the biological tissue is mounted on a frame to form the leaflet structure. It is preferably that the treatment of the biological tissue is carried out on the tissue which is fixed on a frame. This frame is also known as a stent. The stent can be made from a metallic wire frame or a hard plastic such as polyacetal or polyethylene. Preferably, the metallic frame or metallic stent is used. For successful use as a stent, the material has to withstand a degree of mechanical stress and creep.

The shape of the heart valve bioprosthesis corresponds to the patient's native heart valve and aortic root which makes it anatomically tailored for the particular patient. The shape is maintained by creating a specially designed stent which is able to give normal physiological shape and function after implantation or replacement. For a stented pericardial heart valve bioprosthesis an anatomically tailored stent is prepared corresponding to the shape of the patient's native pathological heart valve and aortic root and the tissue of the autologous pericardium is sutured to the stent. The anatomically tailored stent models and the conditioned pericardial tissue makes it easier for the pericardial heart valve replacement to settle in the aortic root improving its function and lifespan.

The stent comprises a stent frame having rows of cells and a proximal section and a distal section at a longitudinal axis of the stent. The stent frame is formed by a plurality of arms, the arms being connected to one another at connection points. The plurality of arms further forms a plurality of diamond-shaped stent cells, in particular the rows of cells, formed of vertices at said connection points between the arms. Furthermore, the stent comprises a dry valve preferably made out bovine pericardium arranged at least at the distal section of the stent with the dry bovine pericardium being configured to be rehydrated with a solution. Also, a skirt surrounding the dry valve and comprising at least one of bovine pericardium and polyester is provided. Additionally, the stent comprises one or more eyelets arranged at a distal end of some of the arms, with the eyelets being configured to fix the valve to the stent frame.

In this connection it is noted that distal in the framework of this application means a part that is most distant from the center of the human heart and proximal means that end that is closest to the center of the human heart.

The stent for an implant or prosthesis described herein is in particular suitable for the treatment of heart valve disease via minimally invasive transcatheter implantation to replace a defective valve.

The stent comprises a plurality of arms which build the stent frame with two sections, i.e. the proximal section and the distal section. The expansion of the stent is made possible by the plurality of arms that are interconnected in such a way that following the expansion they adapt to the anatomical need of the location of the stent. In this way the design of such a stent is adapted to accommodate the anatomical needs and implantation locations. The stent furthermore consists of an expandable, in particular a balloon-expandable, stent frame. Moreover, between 6 and 50 arms can be provided to form the stent frame.

Therefore, the plurality of arms is connected to one another at connection points and form a plurality of diamond-shaped stent cells, in particular the rows of the stent cells, formed of vertices at said connection points between the arms. In this connection it should be noted that the term vertex refers to a corner region of each cell, i.e. the region of the cell forming a corner where two respective sections of the arms meet. Preferably the vertex at least substantially forms an origin of the corner where two respective sections of the arms meet.

It should further be noted in this connection that the sections of the plurality of arms forming sides of the stent cells are linear, such that the formed stent cells comprise a diamond shape. Such shapes can be manufactured in a simple manner and provide the stent frame with an increased stability and flexibility.

Due to the comparably short length of the stent, i.e. 1 to 10 cm, preferably 1.5 to 6 cm, the implant comprising such a stent can be placed more accurately within a blood vessel thereby improving the function of the implant due to the increased apposition to the native annular anatomy using appropriate design options. This is necessary to reduce trauma to a patient during implantation, and to ensure accurate implant placement. The reduction of trauma to a patient also makes the surgery less critical so that this kind of implant can also be provided in patients who were previously not operable due to the too high a risk associated with the implantation of prior art designs.

As described above, the stent preferably comprises a dry valve made of bovine pericardium before treating according to the method of the present invention. The pericardium is used as a heart valve to replace the damaged or diseased naturally occurring heart valve. In that case, the heart valve is firstly attached to the stent, and then is treated step by step beginning with step (1). After the treatment step (4), the step of drying of the biological tissue or a heart valve is carried out using the stent with fixed heart valve.

The heart valves used as a bioprosthesis may be judged by the clinical complications after the implantation. Therefore, it is of significant importance to carry out different tests on valve functionality before the valves are treated and prepared for the surgical and transcatheter replacement or repair. These tests provide empirical evidence for the continued structural integrity of heart valves. Some of tests are designed to simulate the physiological strain due to the expansion and contraction of the surrounding vessel or the movement of the leaflet. Other tests are used to evaluate a heart valve fatigue such as leaflet durability by wear test, leaflet kinematics by evaluating opening and closing behavior of the valve, valve hydrodynamic performance inspections, optical inspection of valves in terms of visual and SEM inspections or high-speed video inspection.

As already mentioned above, the valves are preferably made using bovine pericardium. The ECM tissue is generally harvested from the pericardial sac of cows and is then used to manufacture the leaflets. The tissue from pericardial sac is particularly well suited for a valve leaflet due to its durable physical properties. The tissues are glutaraldehyde fixed, non-viable, chemically treated (decellularized) and sterilized so that the biological markers are removed making them more compatible with the patient's immune system. Therefore, the method of treating the biological tissue according to the present invention may be also named as a method of decellulizing the biological tissue.

In a second aspect, the present invention is directed at a method of treating biological tissue comprising the steps of:
(1) providing bovine or porcine pericardium as the biological tissue as a heart valve;
(2) providing a stent frame;
(3) attaching the biological tissue to the stent frame;
(4) testing the functionality of the biological tissue at the stent frame; and
(5) carrying out a method of decellulizing the biological tissue after step (4).

Preferably, the method of decellulizing the biological tissue comprises the steps of:
(1) contacting the biological tissue with an aqueous solution of Hydrogen Peroxide;
(2) contacting the biological tissue with an aqueous solution of Sodium Hydroxide;
(3) contacting the biological tissue with an aqueous solution of Triton, SDS and EDTA;
(4) contacting the biological tissue with a glycerol solution.

The description regarding the biological tissue, a stent frame, testing the functionality as well as a method of decellulizing comprising the steps of the method of treating biological tissue of the first aspect of the present invention, can be found in details in the part relating to the first aspect of the present invention.

In a third aspect, the present invention relates to a biological tissue comprising a biological tissue prepared according to the method of the first and/or the second aspect as described above.

The following examples are for purpose of illustration only and are not intended to limit the scope of the present invention as defined in the claims which are appended hereto:

### COMPARATIVE EXAMPLE 1-1

40 membranes with 1x1cm of a biological tissue selected from bovine pericardium "sample 180702-1" (P+F Brasil, EDQM certified) has been at first removed from 0.625% glutaraldehyde solution (P+ F GmbH/ Biocollagen) and after that soaked in cold 0.9% saline solution (JP Pharma) for 3 minutes. The soaked tissue has then been immersed in Hydrogen Peroxide 0.5% per volume (Sigma-Aldrich) at 18°C for 60 minutes. As a next step, the tissue was contacted with cold (10°C) PBS pH 7.4 and 0.5% by weight EDTA for 3 minutes (Sigma-Aldrich). After that the tissue was immersed in 99% per volume ethanol (Sigma-Aldrich) at 22°C temperature for 60 seconds with intense stirring, and then immersed in a mixture of glycerol/ethanol (50/50) and 0.5% by weight EDTA (Sigma-Aldrich) at 22°C temperature for 60 minutes with slow stirring. Further, the tissue was immersed in glycerol 99% (Sigma-Aldrich) at 22°C temperature for 120 minutes with slow stirring. Next, the tissue was soaked with absolute ethanol 99% per volume (Sigma-Aldrich) and 0.5% by weight EDTA at 22°C temperature during 60 seconds with intense stirring. Finally, the tissue undergoes a procedure of solvent evaporation under HEPA filtered air insufflation during 18 hours at 18°C temperature. Then, the tissue was packed at double Tyvek pouch and ETO sterilized (55°C, 4 hours ETO exposition) at Sterium Company.

### COMPARATIVE EXAMPLE 1-2

40 membranes with 1x1 cm of a biological tissue selected from bovine pericardium "sample 180702-2" has been prepared according to procedure described in Example 1-1.

### COMPARATIVE EXAMPLE 1-3

40 membranes with 1x1cm of a biological tissue selected from bovine pericardium "sample 180803-1" has been removed from 0.625% glutaraldehyde solution (P+ F GmbH/ Biocollagen) and used as a comparative sample without further treatment.

### EXAMPLE 1-4

Bovine pericardium membranes from **Example 1 and Comparative Examples 1-1 to 1-3** (number of samples: Example 1 is according to the present invention, and 180702-2, 180702-1 and 180803-1 are comparative examples) were soaked in saline solution for 2 minutes and then immersed in a Simulated Body Fluid (SBF), which is a solution with ionic concentration similar to that of human blood plasma such as demonstrated in Table 1, maintained under the same physiological conditions of pH and temperature (pH 7.4 and temperature of 36.5°C). The immersion time varied between 1 and 30 days.

**TABLE 1**

| | Na⁺ | K⁺ | Ca²⁺ | Mg2+ | HCO₃²- | CI- | HPO₄²⁻ | SO₄²⁻ |
|---|---|---|---|---|---|---|---|---|
| SBF (mg/L) | 213.0 | 7.5 | 3.8 | 2.3 | 6.3 | 223.0 | 1.5 | 0.75 |

The samples were digested in a closed flask conductive heating system called CHDS. The calibration curve was prepared from the Specsol^{®} 1000 mg. L⁻¹ standard solution to contain 0.0 - 20 mg. L⁻¹ Ca.

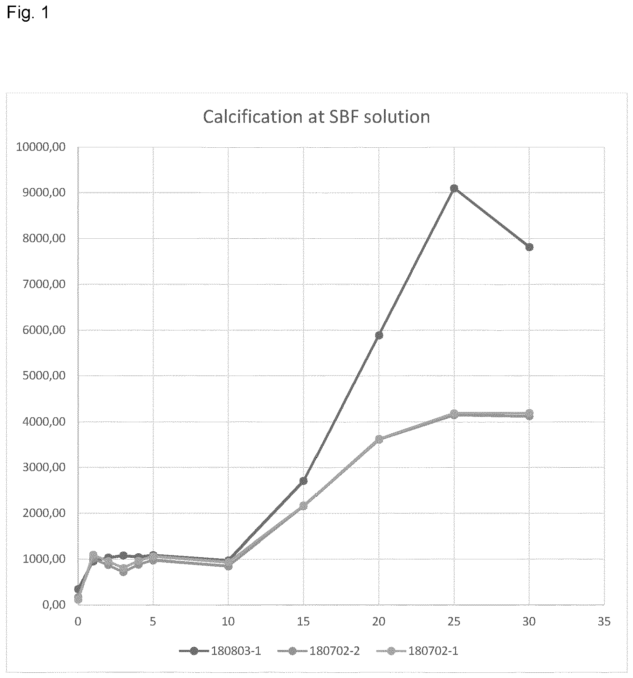

The determination of Ca presented in Fig. 1 was performed on a ContrAA 300 High Resolution Continuous Source Atomic Absorption Spectrometer (HR-CS FAAS) (Analytic Jena, Jena, Germany), equipped with a short arc lamp of Xe as a continuous source of radiation (in Fig. 1 the sign "," on Y-axis has to be read as a point according to Table 2, i.e. the value 1000,00 has to be understood as 1000 or 1000.00).

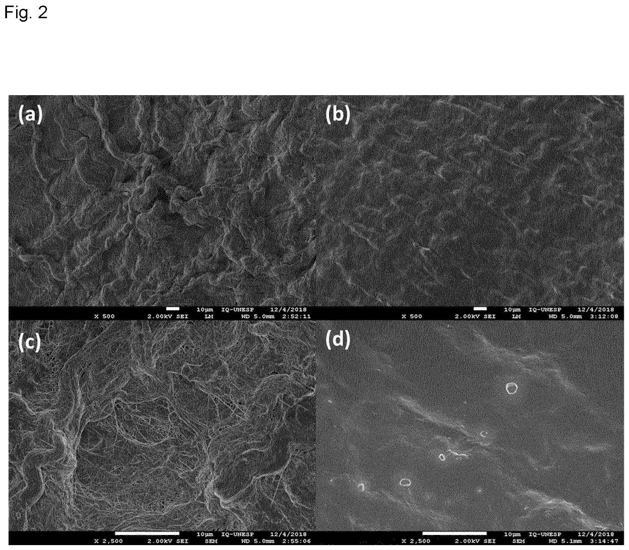

Scanning electron microscope (SEM) was performed on the samples of bovine pericardium (180803-1 glutaraldehyde preserved) using afield emission scanning electron microscope (FESEM) (JEOL, model 7500F): (a) and (c) without SBF, (b) and (d) immersed in SBF solutions for 24 hours (see Fig. 2).

## Claims

1. A method of treating biological tissue comprising the steps of:
(1) contacting the biological tissue with an aqueous solution of Hydrogen Peroxide;
(2) contacting the biological tissue with an aqueous solution of Sodium Hydroxide;
(3) contacting the biological tissue with an aqueous solution of Triton, SDS and EDTA;
(4) contacting the biological tissue with a glycerol solution.

2. The method of claim 1, wherein the biological tissue is fixed with glutaraldehyde before treating.

3. The method of claim 1 or 2, wherein the method further comprises steps (2a) and/or (3a) of contacting the biological tissue with an aqueous solution of TRIS and EDTA.

4. The method according to any one of claims 1 to 3, wherein the method further comprises steps (3b) and/or (4a) of contacting the biological tissue with ethanol within a concentration of at least 70% by volume.

5. The method according to any one of claims 1 to 4, wherein the biological tissue is bovine or porcine pericardium or a heart valve.

6. The method according to any one of claims 1 to 5, wherein hydrogen peroxide in step (1) has a concentration of 1% to 30% by volume.

7. The method according to any one of claims 1 to 6, wherein sodium hydroxide in step (2) has a concentration of 0.1M to 10M.

8. The method according to any one of claims 1 to 7, wherein in steps (2a) and (3a) a concentration of TRIS is of 1mM to 30mM and a concentration of EDTA is of 0.1mM to 5mM.

9. The method according to any one of claims 1 to 8, wherein in step (3) a concentration of Triton is of 0.1% to 10.0% by volume, a concentration of SDS is of 0.05% to 5.0% by volume and a concentration of EDTA is of 0.05mM to 5mM.

10. The method according to any one of claims 1 to 9, wherein the treatment is carried out on the biological tissue which is fixed on a metallic frame.

11. The method according to any one of claims 1 to 10, wherein the method further comprises the step (5) of drying the biological tissue.

12. The method according to any one of claims 1 to 11, wherein the biological tissue is a heart valve for placement at a stent.

13. The method according to claim 11 and claim 12, wherein the step (5) of drying is carried out after fixing the heart valve to the stent.

14. The method according to claim 12 or claim 13, wherein the heart valve is attached to the stent prior to step (1).

15. The method according to claim 14, further comprising the step of testing the valve functionality prior to step (1).

16. The method according to any one of claims 1 to 15, wherein the method of treating biological tissue is a method of decellulizing the biological tissue.

17. A method of treating biological tissue comprising the steps of:
(1) providing bovine or porcine pericardium as the biological tissue as a heart valve;
(2) providing a stent frame;
(3) attaching the biological tissue to the stent frame;
(4) testing the functionality of the biological tissue at the stent frame; and
(5) carrying out a method of decellulizing the biological tissue after step (4).

18. The method according to claim 17, wherein the method of decellulizing the biological tissue comprises the steps of:
(1) contacting the biological tissue with an aqueous solution of Hydrogen Peroxide;
(2) contacting the biological tissue with an aqueous solution of Sodium Hydroxide;
(3) contacting the biological tissue with an aqueous solution of Triton, SDS and EDTA;
(4) contacting the biological tissue with a glycerol solution.

19. A biological tissue comprising a biological tissue prepared according to any one of claims 1 to 18.
